# EUROPEAN PATENT APPLICATION

(11) **EP 0 569 139 A2**
(43) Date of publication of application: **10.11.1993**
(21) Application number: 93302852.4
(22) Date of filing: 13.04.1993
(51) Int. Cl.: C07C 205/56, C07C 205/11, C07C 205/34, C07C 205/06, C07C 255/35, C07D 317/00

(54) **Alkyl substituted nitrotoluene derivatives**

(30) Priority: 07.05.1992 GB 9209872
(71) Applicant: ZENECA LIMITED, London W1Y 6LN (GB)
(72) Inventor: Milner, David John, Whitefield, Manchester M25 7LW (GB)
(74) Representative: Giles, David Eric

(57) **Abstract**

A compound of Formula (1): 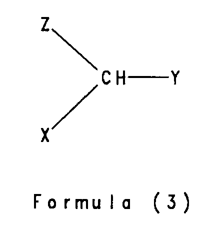
wherein:
R is -H or alkyl;
R¹, R² and R³ each independently is -H or alkyl;
X is halogen, -SO₂alkyl, -CN, -CO₂alkyl, -CO₂alkylaryl, -CO₂aryl, -CO₂H or aryl; and
Y is H, alkyl, alkoxy, aryl or halogen, and a process for their preparation are disclosed.

The compounds of Formula (1) are useful as intermediates in the preparation of dyestuffs, pharmaceuticals and agrochemicals.

## Description

This invention relates to 3-alkyl and 3,5-dialkyl substituted 4-nitrotoluene derivatives and to a method for their preparation.

Reactions of nitroarenes with dihaloethanoates under alkaline conditions have been described in Synthesis, 850(1990) and with 2-chloropropionate esters under alkaline conditions in J. Org. Chem., 49, 578(1984). It is known that under alkaline conditions an alkyl group ortho or para to a nitro group in an aromatic compound is activated and carbanion formation is promoted leading to condensation reactions such as Claisen condensations (Ber. , 30, 1030(1987)). Formation of carbanions from alkyl nitrobenzenes gives condensation products as disclosed by Lapworth (J. Chem. Soc. 1275, (1901)) and these self condensation reactions in alkaline conditions have been used to prepare 1,2-(dinitrodiaryl)ethanes (Bull. Soc. Chim. France, 2444 (1972)). There are no reports of the reaction of alkylnitroarenes with haloalkanoates presumably because the former compounds are expected to readily self condense and form dimeric compounds in alkaline conditions.

The object of the present invention is to provide 2-alkyl, 3-alkyl and 3,5-dialkylnitrotoluene derivatives and a convenient process for their preparation.

According to the present invention there is provided a compound of Formula (1): 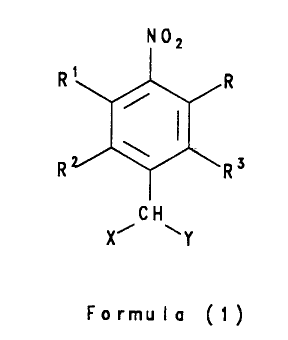
wherein:
- R: is -H or alkyl;
- R¹, R² and R³: each independently is -H or alkyl;
- X: is halogen, -SO₂alkyl, -CN, -CO₂alkyl, -CO₂alkylaryl, -CO₂aryl, -CO₂H or aryl; and
- Y: is -H, alkyl, alkoxy, aryl or halogen.

In compounds of Formula (1) R is preferably C₁₋₆-alkyl, more preferably C₁₋₄-alkyl and especially methyl, ethyl or isopropyl; each of R¹, R² and R³ is preferably -H or C₁₋₆-alkyl, more preferably -H or C₁₋₄-alkyl and especially -H, methyl or ethyl; X is preferably -Cl, -Br, -F, -SO₂C₁₋₄-alkyl, -CN, -CO₂C₁₋₄-alkyl, -CO₂C₁₋₄-alkylphenyl, -CO₂phenyl, -CO₂H or phenyl, more preferably -Cl, -Br, -SO₂CH₃, -CN, -CO₂benzyl, CO₂phenyl, -CO₂C₁₋₄-alkyl, -CO₂H or phenyl and especially -CO₂C₁₋₄-alkyl; Y is preferably -H, C₁₋₄-alkyl, C₁₋₄-alkyloxy, phenyl, Cl, -Br or -F and more preferably C₁₋₄-alkyl, -Cl or -Br.

A preferred sub group of compounds of Formula (1) is that in which R, R¹ and R² are all -H and R³ is C₁₋₄-alkyl, X is -CO₂C₁₋₄-alkyl and Y is -Cl or C₁₋₄-alkyl.

A further preferred sub group of compounds of Formula (1) is that in which R is C₁₋₄-alkyl, R¹, R² and R³ are all -H, X is -CO₂C₁₋₄-alkyl and Y is -Cl or C₁₋₄-alkyl.

A further preferred sub group of compounds of Formula (1) is that in which R and R¹ each independently is C₁₋₄-alkyl, R² and R³ are both -H, X is -CO₂C₁₋₄-alkyl and Y is-Cl or C₁₋₄-alkyl.

According to a further feature of the present invention there is provided a process of the preparation of a compound of Formula (1) by reacting a compound of Formula (2): 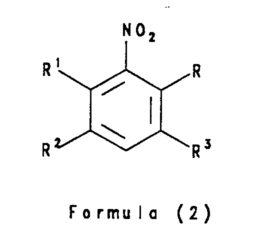
with a compound of Formula (3): 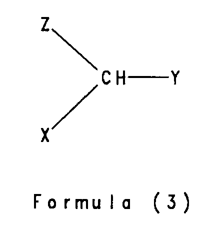
in the presence of an alkali metal C₁₋₄-alkoxide and an aprotic liquid wherein Z is halogen and R, R¹, X and Y are as hereinbefore defined.

The process may be performed by adding a mixture of the compounds of Formulae (2) and (3) to a mixture of the alkali metal alkoxide and an aprotic liquid.

The alkali metal is preferably lithium, sodium or potassium, more preferably sodium or potassium. The C₁₋₄-alkoxide is preferably methoxide, ethoxide, isopropoxide or tertiary butoxide more preferably methoxide. It is further preferred to use a C₁₋₃-alkoxide, especially a methoxide which have the advantage of producing higher yields of desired products. An especially preferred alkali metal alkoxide is sodium methoxide. The aprotic liquid is preferably dimethylformamide, tetrahydrofuran or N-methylpyrrolidone and more preferably dimethylformamide.

The reaction is preferably carried out at a temperature from -60°C to 0°C, more preferably from -50°C to -5°C and especially from -40°C to -10°C.

When the reaction is substantially complete, as judged for example by gas chromatographic analysis,the product may be isolated in any convenient manner, for example, pouring the reaction mixture into aqueous hydrochloric acid, extracting the derived product into an organic liquid, evaporation of the organic liquid and purification of the residual product by fractional distillation or column chromatography (silica).

The compounds of Formula (1) where X is -CO₂H may also be prepared by hydrolysis of the corresponding ester under acidic or basic conditions.

The compounds of the present invention are useful as intermediates in the preparation of dyestuffs, pharmaceuticals and agrochemicals.

The invention is further illustrated by the following examples in which all parts and percentages are by weight:

### Example 1

### Preparation of methyl 2-chloro-2-(3-methyl-4-nitrophenyl)ethanoate

A mixture of 2-nitrotoluene (1.4 parts) and methyl dichloroethanoate (1.6 parts) was added slowly to a stirred mixture of sodium methoxide (1.7 parts, 95%) and dimethylformamide (30 parts) at -14°C. The reaction mixture was poured into aqueous hydrochloric acid (5%, 1000 parts) and extracted with dichloromethane (2x200 parts), the dichloromethane extracts were combined, washed with water and dried over magnesium sulphate. The dichloromethane was removed under vacuum from the dried extracts to leave a residue (1.7 parts). The residue contained methyl 2-chloro-2-(3-methyl-4-nitrophenyl)ethanoate (80%) , 2-nitrotoluene (10%) and 2,2-dinitrobenzyl (3%). The residue was purified by elution from silica using petroleum spirit/dichloromethane as eluent. Evaporation of the solvent gave methyl 2-chloro-2-(3-methyl-4-nitrophenyl)ethanoate the identity of which was confirmed by ¹HNMR.

### Example 2

### Preparation of methyl 2-chloro-2-(3-ethyl-4-nitrophenyl)ethanoate

A mixture of 2-nitroethylbenzene (1.51 parts) and methyl dichloroethanoate (1.57 parts) was added to a stirred mixture of potassium t-butoxide (3.4 parts) in dimethylformamide (30 parts) at -50°C. The reaction mixture was treated as in Example 1 to leave a residue which was purified by elution from silica using firstly petroleum spirit (b.pt. 60-80°C) as eluent, followed by petroleum spirit containing successively larger amounts of dichloromethane as eluent Combination of eluent fractions, based on gas chromatographic analysis, followed by evaporation of solvents gave methyl 2-chloro-2-(3-ethyl-4-nitrophenyl)ethanoate (1.3 parts, 50%). ¹HNMR data confirmed the identity of the product obtained.

### Example 3

### Preparation of ethyl 2-methyl-2-(3-methyl-4-nitrophenyl)ethanoate

The procedure of Example 2 was followed except that 2-nitroluene (1.37 parts) was used in place of 2-nitroethylbenzene, and ethyl 2-chloropropionate (1.45 parts) was used in place of methyl dichloroethanoate and the reaction was carried out at -40°C rather than -50°C.

Ethyl 2-methyl-2-(3-methyl-4-nitrophenyl)ethanoate (1.4 parts, 60%) was obtained and its structure confirmed by ¹HNMR.

### Example 4

### Preparation of methyl 2-chloro-2-(3-methyl-4-nitrophenyl)ethanoate

The procedure of Example 2 was followed except that 3-nitrotoluene (6.9 parts) was used in place of 2-nitroethylbenzene, 7.15 parts instead of 1.57 parts of methyl dichloroethanoate, 17 parts instead of 3.4 parts of potassium t-butoxide and 100 parts instead of 30 parts of dimethylformamide were used and the reaction was carried out at -40°C rather than -50°C.

Methyl 2-chloro-2-(3-methyl-4-nitrophenyl)ethanoate (7.5 parts, 62% was obtained and its structure confirmed by ¹HNMR.

## Claims

1. A compound of Formula (1): 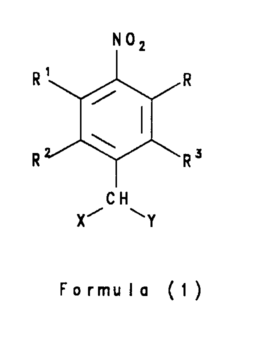 wherein:
R is -H or alkyl;
R¹, R² and R³ each independently is -H or alkyl;
X is halogen, -SO₂alkyl, -CN, -CO₂alkyl, -CO₂alkylaryl, -CO₂aryl, -CO₂H or aryl; and
Y is H, alkyl, alkoxy, aryl or halogen.

2. A compound according to Claim 1 wherein R is C₁₋₆-alkyl; R¹, R² and R³ each independently is H or C₁₋₆-alkyl; X is -Cl, -Br, -F, -SO₂C₁₋₄-alkyl, -CN, -CO₂C₁₋₄-alkyl, -CO₂C₁₋₄-alkylphenyl, -CO₂phenyl, -CO₂H or phenyl; and Y is -H, C₁₋₄-alkyl, C₁₋₄-alkoxy, phenyl, -Cl, -Br or -F.

3. A compound according to Claim 1 wherein R, R¹ and R² are -H; R³ is C₁₋₄-alkyl; X is -CO₂C₁₋₄-alkyl; and Y is -Cl or C₁₋₄-alkyl.

4. A compound according to Claim 1 wherein R is C₁₋₄-alkyl, R¹, R² and R³ are -H; X is -CO₂C₁₋₄-alkyl; and Y is -Cl or C₁₋₄-alkyl.

5. A compound according to Claim 1 wherein R and R¹ each independently is C₁₋₄-alkyl, R² and R³ are -H; X is -CO₂C₁₋₄-alkyl and Y is -Cl or C₁₋₄-alkyl.

6. A process of the preparation of a compound of Formula (1) by reacting a compound of Formula (2): 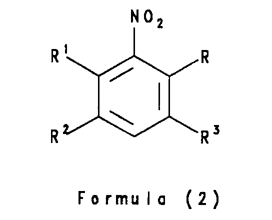 with a compound of Formula (3): 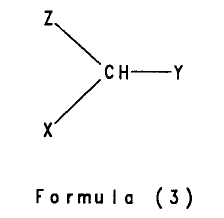 in the presence of an alkali metal C₁₋₄-alkoxide and an aprotic liquid wherein:
R is -H or alkyl;
R¹, R² and R³ each independently is H or alkyl;
X is halogen, -SO₂alkyl, -CN, -CO₂alkyl, -CO₂alkylaryl, -CO₂aryl, -CO₂H or aryl;
Y is H, alkyl, alkoxy, aryl or halogen; and
Z is halogen.
